(19) Europäisches Patentamt
European Patent Office
Office européen des brevets

(11) **EP 3 892 857 A2**

(12) **EUROPEAN PATENT APPLICATION**

(43) Date of publication:
**13.10.2021 Bulletin 2021/41**

(51) Int Cl.:
**F04B 49/06** *(2006.01)*

(21) Application number: **21167197.9**

(22) Date of filing: **07.04.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.04.2020 JP 2020070045**

(71) Applicant: **Sumitomo Rubber Industries, Ltd.**
**Kobe-shi, Hyogo-ken 651-0072 (JP)**

(72) Inventor: **NAGAHAMA, Masamune**
**Kobe-shi, Hyogo-ken, 651-0072 (JP)**

(74) Representative: **Manitz Finsterwald**
**Patent- und Rechtsanwaltspartnerschaft mbB**
**Martin-Greif-Strasse 1**
**80336 München (DE)**

(54) **PUMP SYSTEM**

(57)      Provided is a pump system including: a pump configured to send out a fluid in response to application of a voltage, an ejection amount of the fluid increasing or decreasing in accordance with the voltage; a flow meter configured to measure a flow rate of the fluid sent out from the pump; and a control unit connected to the pump and the flow meter, and configured to control the voltage that is applied to the pump according to the flow rate measured by the flow meter.

EP 3 892 857 A2

**Description**

The disclosure relates to a pump system.

JP 2011-160868A discloses a pump using a MEMS (micro electro mechanical systems) technique. According to JP 2011-160868A (hereinafter called "Patent Literature 1"), in order to control the flow rate of the pump, the actual flow rate of a fluid sent out by the pump is detected by a flow rate sensor, and drive pulses for the pump are generated based on the detected flow rate. More specifically, the time at which to turn the pump on and off is controlled through feedback control that performs feedback of the actual flow rate, and thus the flow rate of the pump is controlled.

However, when attempting to control the flow rate of a pump by controlling only the time at which to turn the pump on and off as in Patent Literature 1, the flow of the fluid is not uniform and becomes non-continuous due to the intermittent operation of the pump. In this case, the level of precision of the flow rate sensor in measuring the flow rate may decrease, which makes it impossible to precisely control the flow rate of the pump.

It is an object of the disclosure to provide a pump system capable of generating a more uniform flow of a fluid and precisely controlling the flow rate of a pump.

A pump system according to a first aspect is including a pump configured to send out a fluid in response to application of a voltage, an ejection amount of the fluid increasing or decreasing in accordance with the voltage, a flow meter configured to measure a flow rate of the fluid sent out from the pump, and a control unit connected to the pump and the flow meter, and configured to control the voltage that is applied to the pump according to the flow rate measured by the flow meter.

A pump system according to a second aspect is the pump system according to the first aspect, in which a target flow rate of the pump is 600 μl/h or less.

A pump system according to a third aspect is the the pump system according to the first or second aspect, wherein the control unit adjusts a magnitude of the voltage so as to increase the ejection amount in a case in which the flow rate measured by the flow meter is smaller than a target flow rate of the pump, and adjusts a magnitude of the voltage so as to reduce the ejection amount in a case in which the flow rate is larger than the target flow rate.

According to the above-described aspects, the flow rate of a pump is measured by a flow meter, and feedback of the measured flow rate is performed to control a voltage, which is a control parameter for increasing or decreasing the ejection amount of the pump. That is to say, the flow rate of the pump is controlled by increasing or decreasing the ejection amount of fluid that is sent out from the pump at a time. Accordingly, a more uniform flow of a fluid can be generated, and the level of measurement precision of the flow meter is improved, which makes it possible to precisely control the flow rate of the pump.

Further embodiments of the invention are described in the following description of the figures and/or the dependent claims attached to this specification. The invention will be explained in the following in detail by means of embodiments and with reference to the drawing in which is shown:

FIG. 1 is a configuration diagram of a pump system according to an embodiment of the disclosure.
FIG. 2A is a side cross-sectional view of the pump during suction.
FIG. 2B is a side cross-sectional view of the pump during ejection.
FIG. 3 is a flowchart showing the flow of feedback control that controls the ejection amount of the pump based on a measured value of a flow meter.
FIG. 4 is a graph showing a waveform of a voltage that is applied to the pump.
FIG. 5 is a configuration diagram of a pump system according to a modified example.

In the following the same reference numerals will be used for parts having the same or equivalent function. Any statements made having regard to the direction of a component are made relative to the position shown in the drawing and can naturally vary in the actual position of application.

Hereinafter, a pump system according to an embodiment of the disclosure will be described with reference to the drawings.

1. Configuration of Pump System

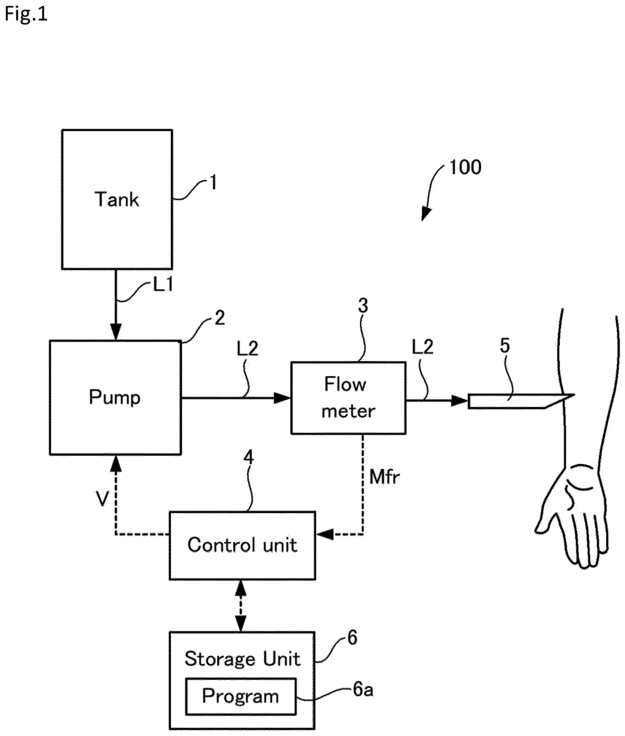

FIG. 1 shows a configuration diagram of a pump system 100 according to this embodiment. As shown in FIG. 1, the pump system 100 includes a tank 1 configured to store a fluid, and a pump 2 arranged downstream of the tank 1 and configured to suck and eject the fluid in the tank 1. In this description, the upstream and the downstream are defined according to the flow of a fluid. Furthermore, the pump system 100 further includes a flow meter 3 configured to measure the flow rate of the fluid sent out from the pump 2, and a control unit 4 connected to the pump 2 and the flow meter 3. The control unit 4 controls the fluid transporting operation that is performed by the pump system 100. At this time, the control unit 4 performs feedback control that controls the ejection amount (flow rate) of the pump 2, based on a measured value Mfr of the flow rate measured by the flow meter 3.

The pump 2 of this embodiment is, but is not limited to, a small or ultra-small pump that enables a fluid to flow at a very low flow rate using a MEMS (micro electro mechanical systems) technique. Quantitatively, the pump 2 may be a small pump whose target flow rate is set to 600 μl/h or less. However, the pump 2 may also be a smaller pump whose target flow rate is set to 400 μl/h or less, 200 μl/h or less, 100 μl/h or less, 80 μl/h or less, 60 μl/h or less, 40 μl/h or less, or 20 μl/h or less.

Furthermore, the pump system 100 of this embodiment is, but is not limited to, a system configured to transport a medical fluid such as insulin. Thus, in this embodiment, a needle 5 is connected downstream of the pump 2, and the medical fluid in the tank 1 is given to a patient by the needle 5 being inserted into a patient's arm.

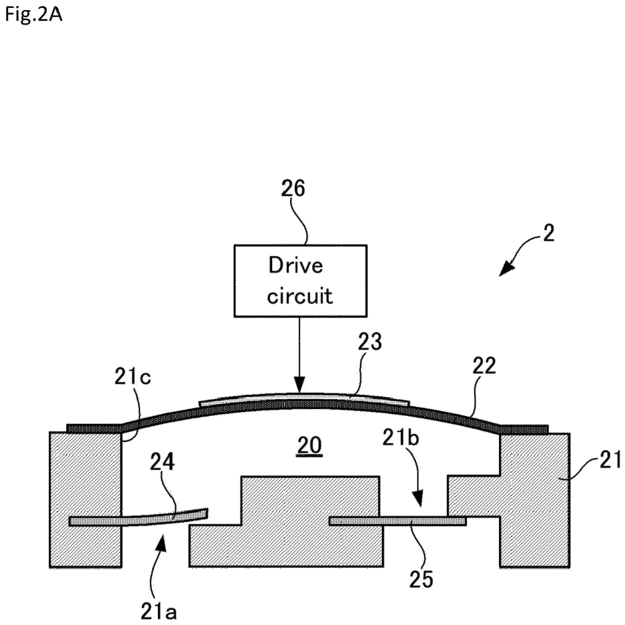

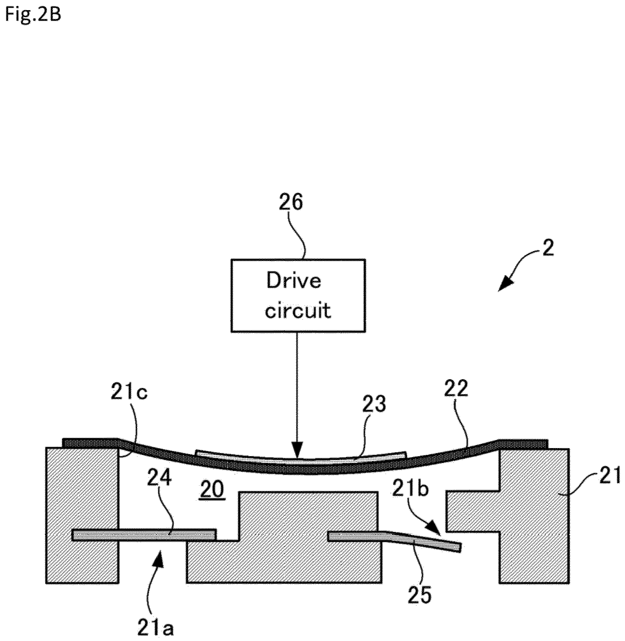

Furthermore, the pump 2 of this embodiment is, but is not limited to, a piezo pump. FIGS. 2A and 2B are side cross-sectional views of the pump 2 illustrating an operating principle of the pump 2. As shown in FIGS. 2A and 2B, the pump 2 includes a casing 21, a diaphragm 22, and a piezo element 23. A pump chamber 20 is arranged inside the casing 21, and a suction port 21a and an ejection port 21b are connected to the pump chamber 20 in the casing 21. A suction valve 24 configured to open and close the suction port 21a is attached to the port, and an ejection valve 25 configured to open and close the ejection port 21b is attached to the port. Furthermore, an opening 21c is arranged passing through the casing 21, and the diaphragm 22 is attached to the casing 21 so as to close the opening 21c.

The piezo element 23 is attached to the diaphragm 22. The pump 2 further includes a drive circuit 26 configured to drive the piezo element 23. The drive circuit 26 vibrates the piezo element 23, thereby vibrating the diaphragm 22 up and down. The piezo element 23 is constituted by a thin film layer made of a piezoelectric material, and a pair of electrodes respectively arranged on a pair of end faces of the thin film layer, and a voltage V is applied from the drive circuit 26 to a portion between the electrodes. The drive circuit 26 is connected to the control unit 4, and the voltage V that is applied via the drive circuit 26 to the piezo element 23 is controlled by the control unit 4.

When the piezo element 23 is vibrated to deform the diaphragm 22 so as to increase the volume of the pump chamber 20 as shown in FIG. 2A, the pressure inside the pump chamber 20 decreases, and thus the suction valve 24 is opened by being pulled into the pump chamber 20. Accordingly, the fluid in the tank 1 is sucked out via the suction port 21a into the pump chamber 20. The suction port 21a is connected to a first flow path LI, and is further connected via the first flow path L1 to the tank 1.

On the other hand, when the piezo element 23 is vibrated to deform the diaphragm 22 so as to reduce the volume of the pump chamber 20 as shown in FIG. 2B, the pressure inside the pump chamber 20 increases, and thus the ejection valve 25 is opened by being pushed to the outside of the pump chamber 20. Furthermore, at this time, the suction valve 24 is closed by being pushed so as to close the suction port 21a. Accordingly, a fluid is ejected from the pump chamber 20 via the ejection port 21b. The ejection port 21b is connected to a second flow path L2, and the fluid ejected from the pump chamber 20 flows to the downstream side via the second flow path L2 and ultimately reaches the needle 5. Note that, when the piezo element 23 is next vibrated as shown in FIG.

2A, the suction valve 24 is opened, and the ejection valve 25 is closed by being pulled so as to close the ejection port 21b.

The amplitude of the piezo element 23 depends on the voltage V that is applied to the piezo element 23. Accordingly, the amplitude of the diaphragm 22 is decided on according to the voltage V, and the amount of fluid sent from the pump 2, that is, the ejection amount from the pump 2 per a single instance of vibration of the diaphragm 22 is eventually decided on. That is to say, the voltage Visa control parameter for increasing or decreasing the ejection amount from the pump 2 per a single instance of vibration of the diaphragm 22.

Furthermore, the vibration frequency of the piezo element 23 is controlled by controlling a drive frequency f of the piezo element 23. The drive frequency f is also controlled by the control unit 4 via the drive circuit 26. Thus, the drive frequency f is a control parameter for deciding on the vibration frequency of the piezo element 23, and ultimately deciding on the flow rate of the pump 2.

The flow meter 3 of this embodiment is, but is not limited to, a flow meter using a MEMS technique. The flow meter 3 is arranged on the second flow path L2, and measures the flow rate of the fluid that is sent out from the pump 2 and flows through the second flow path L2. Furthermore, the flow meter 3 of this embodiment is a thermal flow meter. Note that the level of precision of the flow meter 3 in measuring the flow rate is kept relatively high in a state in which a uniform flow of a fluid is formed, but the level of precision may decrease in a state in which a non-continuous flow of a fluid is formed due to an intermittent operation of the pump 2 or the like. However, in this embodiment, as will be described later, the magnitude of the voltage V that is applied to the pump 2 is controlled so as to be increased or decreased, and the ejection amount from the pump 2 per a single instance of vibration of the diaphragm 22 is also controlled so as to be increased or decreased. As a result, a more uniform flow of a fluid can be generated, and the level of measurement precision of the flow meter 3 is improved.

The control unit 4 is a microcomputer, and is constituted by a CPU, a ROM, a RAM, and the like. The pump system 100 further includes a non-volatile storage unit 6 connected to the control unit 4. The control unit 4 reads and executes a program 6a stored in the storage unit 6, thereby performing the above-described operations. Note that part or the whole of the program 6a may be stored in a ROM constituting the control unit 4.

A target flow rate Tfr of the pump 2 can be set for the pump system 100. The set target flow rate Tfr is stored in the storage unit 6, and is referred to by the control unit 4 as appropriate. There is no particular limitation on the method for setting the target flow rate Tfr of the pump 2, and, for example, it is also possible that an input device such as a button or a switch is mounted in the pump system 100, and a user can input the target flow rate Tfr by operating the device. Alternatively, it is also possible that the control unit 4 is connectable to an external computer, and the target flow rate Tfr input by the user to the computer is transmitted from the computer to the control unit 4 and stored in the storage unit 6. It is assumed that examples of the computer typically include a smartphone, a tablet computer, a desktop computer, and a laptop computer. There is no particular limitation on the form of communicative connection between the computer and the control unit 4, but it may be a wired connection using a cable or wireless connection according to a wireless communication standard such as Bluetooth (registered trademark).

2. Operation of Pump System

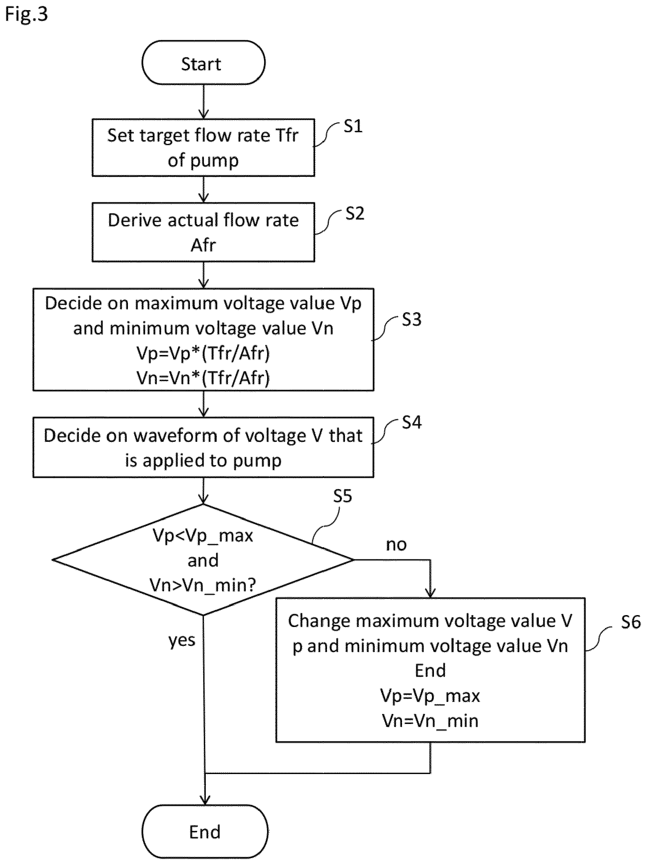

Next, a fluid transporting operation that is performed by the pump system 100 will be described in detail. FIG. 3 is a flowchart showing the flow of feedback control that controls the ejection amount (flow rate) of the pump 2 based on the measured value Mfr of the flow meter 3, the feedback control being performed during the transporting operation.

First, when driving the pump system 100, the user sets the target flow rate Tfr of the pump 2, for example, by operating an input device mounted in the pump system 100 or an external computer connected to the pump system 100, and stores the target flow rate in the storage unit 6. Furthermore, the user inputs a drive command to drive the pump system 100 to the control unit 4, for example, by operating the input device or the external computer.

Upon receiving input of the above-mentioned drive command, the control unit 4 reads the target flow rate Tfr of the pump 2 from the storage unit 6 (see step S1 of FIG. 3). Next, the control unit 4 decides on the drive frequency f of the pump 2. The drive frequency f may be predetermined, or may be calculated from the target flow rate Tfr. There is a constant relationship between the drive frequency f and the target flow rate Tfr. Accordingly, in the case of the latter, the control unit 4 decides on the drive frequency f through matching with the target flow rate Tfr with reference to the information indicating this relationship that is predetermined and stored in the storage unit 6.

Furthermore, the control unit 4 decides on an initial value of the voltage V that is to be applied to the pump 2. In this embodiment, the initial value of the voltage V is predetermined for each target flow rate Tfr, and is stored in the storage unit 6. The control unit 4 reads the value corresponding to the target flow rate Tfr from the storage unit 6, and sets the value as the initial value of the voltage V.

Incidentally, the voltage V changes within the range between a minimum voltage value Vn on the negative side and a maximum voltage value Vp on the positive side according to a predetermined rule that is set in advance, in one suction and ejection section of the pump 2, that is, one vibration section of the diaphragm 22 (which may be hereinafter referred to as a "pumping unit period"). Accordingly, the above-mentioned initial value of the voltage V means the waveform of the voltage V in a first pumping unit period. The predetermined rule will be described later.

The control unit 4 drives the piezo element 23 using the drive circuit 26, based on the initial value of the voltage V and the drive frequency f decided on as described above. After driving the piezo element 23 first, the control unit 4 continuously drives the piezo element 23. Furthermore, each time the control unit 4 drives the piezo element 23, the control unit simultaneously drives the flow meter 3 as well, and causes the flow meter 3 to continuously measure the flow rate of the fluid sent out from the pump 2. The measured value Mfr of the flow rate measured by the flow meter 3 is sequentially transmitted from the flow meter 3 to the control unit 4. Each time the control unit 4 receives the measured value Mfr from the flow meter 3, the control unit derives an actual flow rate Afr of a fluid sent out from the pump 2 according to the measured value Mfr (see step S2 of FIG. 3). In this embodiment, a flow rate Afr is calculated by averaging the measured value Mfr from when the driving is started or from a point in time that is earlier than the measurement time by a predetermined period. Note that the flow rate Afr in this case is derived from the measured value Mfr although it is an average of the measured value Mfr from when the driving is started or from a point in time that is earlier than the measurement time by a predetermined period, and thus it can be said that this is the flow rate measured by the flow meter 3.

The control unit 4 controls the voltage V that is applied to the pump 2, according to the flow rate Afr measured by the flow meter 3. That is to say, each time the control unit 4 derives the flow rate Afr, the control unit decides on the waveform of the voltage V in a next pumping unit period according thereto, and applies the voltage V with the decided waveform to the piezo element 23 in the next pumping unit period. Accordingly, the magnitude of the vibration of the diaphragm 22, that is, the ejection amount from the pump 2 in the next pumping unit period is controlled according to the flow rate Afr measured by the flow meter 3.

More specifically, each time the control unit 4 derives the flow rate Afr, the control unit decides on the maximum voltage value Vp and the minimum voltage value Vn in the next pumping unit period, following the flow rate Afr and the target flow rate Tfr using Equation below (see step S3 of FIG. 3). The current values of Vp and Vn (Vp and Vn in the latest pumping unit period) are substituted for Vp and Vn of the right-hand side in Equation below, and thus Vp and Vn in the next pumping unit period are calculated. Note that the minimum voltage value Vn takes a minus value.

$$Vp = Vp*(Tfr/Afr)$$

$$Vn = Vn*(Tfr/Afr)$$

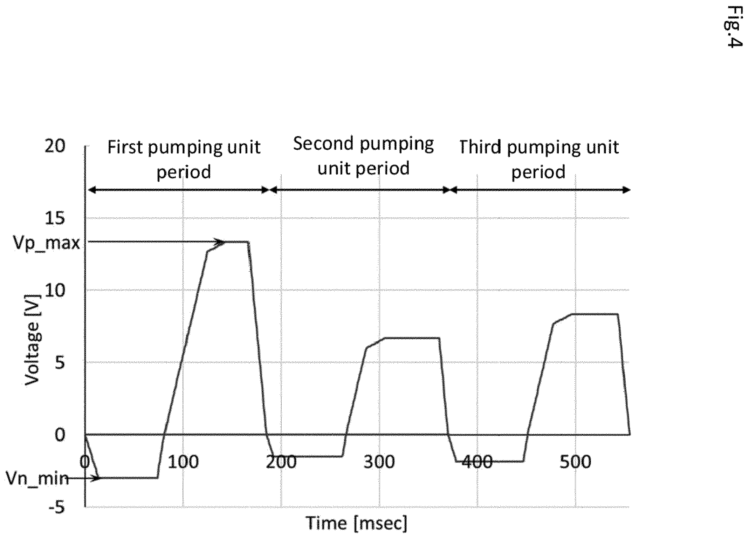

Each time the control unit 4 decides on the maximum voltage value Vp and the minimum voltage value Vn, the control unit decides on the waveform of the voltage V in the next pumping unit period (see step S4 of FIG. 3). FIG. 4 is a graph showing an example of the waveform of the voltage V that is applied to the pump 2, and shows the waveform of the voltage V in three pumping unit periods including the first pumping unit period. In each pumping unit period, the voltage V starts from 0, decreases at a predetermined constant slope C1 to the minimum voltage value Vn, and then is maintained at the minimum voltage value Vn for a certain period of time. Next, the voltage V increases at another predetermined constant slope C2 to 0, and further increases at a predetermined constant slope C3. Subsequently, the voltage increases at another predetermined constant slope C4 for a predetermined period of time T1 to the maximum voltage value Vp. Subsequently, the voltage V is maintained at the maximum voltage value Vp for a certain period of time, and then decreases at another predetermined constant slope C5 to 0. The slopes C1 to C5 and the period of time T1 are shared by different pumping unit periods. Meanwhile, the periods of time during which the minimum voltage value Vn and the maximum voltage value Vp are maintained are different from pumping unit period to pumping unit period, and are decided on by the control unit 4 as appropriate. The control unit 4 decides on the waveform of the voltage V based on the maximum voltage value Vp and the minimum voltage value Vn according to the above-described rule.

Note that, in order to prevent malfunction of the pump 2, an upper limit value Vp_max is predetermined for the maximum voltage value Vp, and a lower limit value Vn_min is predetermined for the minimum voltage value Vn, the limit values being stored in the storage unit 6. Accordingly, after calculating the maximum voltage value Vp and the minimum voltage value Vn in step S3, the control unit 4 compares the maximum voltage value Vp and the upper limit value Vp_max, and further compares the minimum voltage value Vn and the lower limit value Vn_min (see step S5 of FIG. 3). As a result of the comparison, if the maximum voltage value Vp is smaller than the upper limit value Vp_max and the minimum voltage value Vn is larger than the lower limit value Vn_min, the control unit 4 uses the waveform of the voltage V decided on in step S4, as the waveform of the voltage V in the next pumping unit period. On the other hand, if the maximum voltage value Vp is at the upper limit value Vp_max or more or if the minimum voltage value Vn is at the lower limit value Vn_min or less, the control unit changes the maximum voltage value Vp and the minimum voltage value Vn in the next pumping unit period respectively to the predetermined upper limit value Vp_max and lower limit value Vn_min (see step S6 of FIG. 3). At this time, the control unit 4 re-calculates the waveform of the voltage V using a similar method to that in step S4, based on the changed maximum voltage value Vp and minimum voltage value Vn, and uses the re-calculated waveform as the waveform of the voltage V in the next pumping unit period.

Note that the above-described initial value of the waveform of the voltage V is, but is not limited to, a waveform in which the maximum voltage value Vp and the minimum voltage value Vn are respectively the upper limit value Vp_max and the lower limit value Vn_min as in the example in FIG. 4.

As is clear from the description above, if the actual flow rate Afr is smaller than the target flow rate Tfr, the magnitude of the voltage V is adjusted such that the ejection amount from the pump 2 increases, and, if the actual flow rate Afr is larger than the target flow rate Tfr, the magnitude of the voltage V is adjusted such that the ejection amount from the pump 2 decreases. More specifically, the smaller the actual flow rate Afr is relative to the target flow rate Tfr, the larger the magnitudes of Vp and Vn in the next pumping unit period are set to be. Furthermore, the larger the actual flow rate Afr is relative to the target flow rate Tfr, the smaller the magnitudes of Vp and Vn in the next pumping unit period are set to be. That is to say, the smaller the actual flow rate Afr is relative to the target flow rate Tfr, the larger the magnitude of the voltage V (the amplitude of the voltage V) in the next pumping unit period is set to be. Furthermore, the larger the actual flow rate Afr is relative to the target flow rate Tfr, the smaller the magnitude of the voltage V (the amplitude of the voltage V) in the next pumping unit period is set to be.

That is to say, each time the control unit 4 derives the flow rate Afr, the control unit compares the flow rate Afr and the target flow rate Tfr, and decides on a next ejection amount from the pump 2 such that the flow rate Afr that is derived next is closer to the target flow rate Tfr. Thus, if the latest flow rate Afr is smaller than the target flow rate Tfr, the control unit 4 sets the magnitude of the voltage V that is applied to the pump 2 next to a larger value such that the next ejection amount of the pump 2 increases. On the other hand, if the latest flow rate Afr is larger than the target flow rate Tfr, the control unit sets the magnitude of the voltage V that is applied to the pump 2 next to a smaller value such that the next ejection amount of the pump 2 decreases.

As described above, during driving of the pump system 100, the ejection amount from the pump 2 is repeatedly adjusted for each pumping unit period, and frequently increases or decreases in accordance with the voltage V that is frequently controlled according to the actual flow rate Afr. That is to say, the pump 2 is configured not to perform an intermittent operation that either sends out a constant ejection amount of fluid or does not send out a fluid at all each time the diaphragm 22 vibrates once, but is configured to send out a various ejection amounts of fluid. Accordingly, a more uniform flow of the fluid can be generated, and the level of measurement precision of the flow meter 3 is improved. As a result, feedback control based on the measured value Mfr of the flow meter 3 is precisely realized, the flow rate of the pump 2 is precisely controlled, and eventually the target flow rate Tfr is precisely achieved.

The fluid sent out from the pump 2 flows through the second flow path L2 and is sent into a patient by the needle 5 being inserted into a patient's body. In this embodiment, the target flow rate Tfr is precisely achieved, and thus it is possible to precisely give a desired amount of fluid to the patient's body.

3. Modified Examples

In the description above, an embodiment of the disclosure has been described, but the disclosure is not limited to the foregoing embodiment, and can encompass various modifications without departing from the gist thereof. For example, the following modifications are possible.

3-1

In the foregoing embodiment, during driving of the pump system 100, the drive frequency f of the pump 2 was not particularly changed after being initially set, and the pump 2 was continuously driven. However, it is also possible that the drive frequency f is changed as appropriate in response to an external input signal or the like during driving of the pump system 100, as with the voltage V. At this time, it is also possible to adjust the drive frequency f so as to continuously stop the pump 2 until a period of time corresponding to one or at least two pumping unit periods passes. Not so much as in the foregoing embodiment, but, in this case as well, it is possible to achieve a uniform flow of a fluid and to realize low power consumption, by controlling the pump 2 to send out a various ejection amounts of fluid in each pumping unit period.

3-2

In the foregoing embodiment, a piezo pump was given as an example of the pump 2, but, for example, various types of pumps whose ejection amount increases or decreases in accordance with the voltage V that is applied thereto, such as an electro-osmotic pump, may be used as the pump 2.

3-3

The fluid that is transported by the pump system 100 is not limited to a liquid, and may also be a gas.

3-4

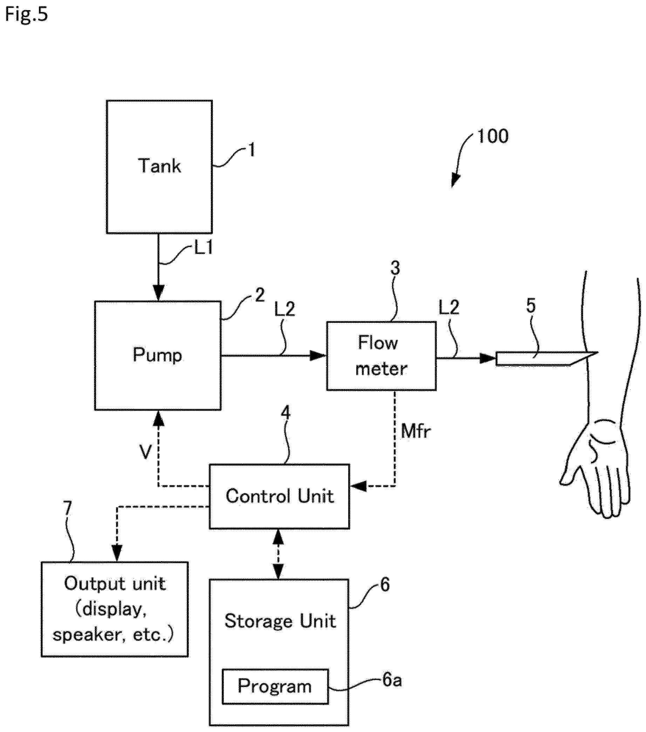

As shown in FIG. 5, an output unit 7 such as a display or a speaker may be mounted in the pump system 100. In this case, it is also possible that the control unit 4 predicts the flow rate of the fluid ejected from the pump 2 and/or the total amount of fluid ejected from the pump 2, based on the voltage V and the drive frequency f during driving of the pump system 100, and visually and/or auditorily outputs an alert via the output unit 7 in the case in which the predicted value is higher than a predetermined reference value. Accordingly, it is possible to notify a user of the possibility that a fluid will be excessively transported (a medical fluid will be excessively given, in the case of giving medicine such as insulin), and to prompt the user to act properly.

LIST OF REFERENCE NUMERALS

100   Pump system
1   Tank
2   Pump
3   Flow meter
4   Control unit
5   Needle
6   Storage unit

**Claims**

1.   A pump system (100) comprising:

a pump (2) configured to send out a fluid in response to application of a voltage, an ejection amount of the fluid increasing or decreasing in accordance with the voltage;
a flow meter (3) configured to measure a flow rate of the fluid sent out from the pump (2); and
a control unit (4) connected to the pump (2) and the flow meter (3), and configured to control the voltage that is applied to the pump (2) according to the flow rate measured by the flow meter (3).

2.   The pump system (100) according to claim 1, wherein a target flow rate of the pump (2) is 600 $\mu$l/h or less.

3.   The pump system (100) according to claims 1 or 2, wherein the control unit (4) adjusts a magnitude of the voltage so as to increase the ejection amount in a case in which the flow rate measured by the flow meter (3) is smaller than a target flow rate of the pump (2), and adjusts a magnitude of the voltage so as to reduce the ejection amount in a case in which the flow rate is larger than the target flow rate.

4.   The pump sytem (100) according to one of claims 1 to 3, wherein the voltage is a control parameter for increasing or decreasing an ejection amount of the pump; and/or wherein the flow rate of the pump is controlled by increasing or decreasing an ejection amount of fluid.

5. The pump system (100) according to one of claims 1 to 4, wherein the pump (2) includes a casing (21), a diaphragm (22), and a piezo element (23).

6. The pump system (100) according to claim 5, wherein a pump chamber (20) is arranged inside the casing (21), and a suction port (21a) and an ejection port (21b) are connected to the pump chamber (20) in the casing (21).

7. The pump system (100) according to claim 5 or claim 5, wherein a suction valve (24) configured to open and close the suction port (21a) is attached to the suction port (21a), and an ejection valve (25) configured to open and close the ejection port (21b) is attached to the ejection port (21b).

8. The pump system (100) according to one of claims 5 to 7, further comprising an opening (21c) is arranged passing through the casing (21), and the diaphragm (22) is attached to the casing (21) so as to close the opening (21c).

9. The pump system (100) according to one of claims 5 to 8, wherein the piezo element (23) is attached to the diaphragm (22).

10. The pump system (100) according to one of claims 5 to 9, wherein the pump (2) further includes a drive circuit (26) configured to drive the piezo element (23).

11. The pump system (100) according to claim 10, wherein the drive circuit (26) is configured to vibrate the piezo element (23).

12. The pump system (100) according to one of claims 5 to 11, wherein the piezo element (23) comprises a thin film layer made of a piezoelectric material, and a pair of electrodes respectively arranged on a pair of end faces of the thin film layer, and wherein a voltage V can be applied from the drive circuit (26) to a portion between the electrodes.

13. The pump system (100) according to one of claims 9 to 12, wherein the drive circuit (26) is connected to the control unit (4), and the voltage (V) that is applied via the drive circuit (26) to the piezo element (23) is controlled by the control unit (4).

14. The pump system (100) according to one of claims 9 to 13, wherein a drive frequency (f) of the piezo element (23) is controlled by the control unit (4) via the drive circuit (26).

15. The pump system (100) according to one of claims 1 to 14, wherein the control unit (4) is a microcomputer, and comprises at least one of the following a CPU, a ROM, a RAM, and the like.

Fig.1

Fig.2A

Fig.2B

Fig.3

Start

Set target flow rate Tfr of pump — S1

Derive actual flow rate Afr — S2

Decide on maximum voltage value Vp and minimum voltage value Vn
Vp=Vp*(Tfr/Afr)
Vn=Vn*(Tfr/Afr) — S3

Decide on waveform of voltage V that is applied to pump — S4

S5

Vp<Vp_max
and
Vn>Vn_min?

no

yes

Change maximum voltage value Vp and minimum voltage value Vn
End
Vp=Vp_max
Vn=Vn_min — S6

End

Fig.4

Fig.5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2011160868 A